# EUROPEAN PATENT SPECIFICATION

(11) **EP 4 279 919 B1**
(45) Date of publication and mention of the grant of the patent: **29.04.2026**
(21) Application number: 22739669.4
(22) Date of filing: 12.01.2022
(51) Int. Cl.: G01N 33/50, G01N 33/68, A61K 35/30, A61P 25/00, C12Q 1/02, C12N 5/0793

(54) **METHOD FOR SCREENING NEURONAL REGENERATION PROMOTING CELLS HAVING NEURONAL REGENERATION ACTIVITY**
VERFAHREN ZUM SCREENING VON NERVENREGENERATIONSFÖRDERNDEN ZELLEN MIT NERVENREGENERATIONSAKTIVITÄT
PROCÉDÉ DE CRIBLAGE DE CELLULES FAVORISANT LA RÉGÉNÉRATION NEURONALE PRÉSENTANT UNE ACTIVITÉ DE RÉGÉNÉRATION NEURONALE

(30) Priority: 12.01.2021 KR 20210003879; 11.01.2022 KR 20220004077
(43) Date of publication of application: 22.11.2023
(73) Proprietor: Cellatoz Therapeutics, Inc., Seongnam-si, Gyeonggi-do 13487 (KR)
(72) Inventor: LIM, Jaeseung, Hwaseong-si Gyeonggi-do 18447 (KR); KIM, Min Young, Seoul 07362 (KR); SUNG, Min Ki, Seoul 06352 (KR)
(74) Representative: V.O.
(86) International application number: PCT/KR2022/000553
(87) International publication number: WO 2022/154474

(56) References cited:
- EP-A2- 2 338 980
- WO-A1-2013/151725
- JP-B2- 4 147 305
- KR-A- 20180 057 614
- US-A1- 2017 258 843
- ANONYMOUS: "A new era of cell therapies for intractable diseases", CELLATOZ THERAPEUTICS, INC. CELLATOZRX.COM/EN, 1 January 2020 (2020-01-01), XP055951544, Retrieved from the Internet <URL:https://media.nature.com/original/magazine-assets/d43747-020-00921-8/d43747-020-00921-8.pdf>

## Description

### [Technical Field]

The present disclosure relates to a method for screening stem cell-derived, neuronal regeneration-promoting cells having neuronal regeneration activity and a pharmaceutical composition for preventing or treating a neurological disease, which contains the neuronal regeneration-promoting cells.

### [Background Art]

Mesenchymal stem cells (MSCs) are used a lot for development of cell therapy agents because they can differentiate into a variety of cell types in response to specific stimulation and are free from the tumorigenicity of induced stem cells and the ethical issues that afflict the use of embryonic stem cells. The mesenchymal stem cells are adult stem cells and are commonly isolated from the tissues of fat, umbilical cord blood, bone marrow, etc. of adults. The methods for isolating these tissues have the problems that they are invasive, induce pain and cannot obtain a large number of stem cells.

### [Technical Problem]

The inventors of the present disclosure have derived a method for screening neuronal regeneration-promoting cells that exhibit neuronal regeneration effect from among various cells differentiated from mesenchymal stem cells by analyzing specific CD markers.

The present disclosure is directed to providing a method for screening mesenchymal stem cell-derived, neuronal regeneration-promoting cells having neuronal regeneration activity.

The present disclosure is also directed to providing neuronal regeneration-promoting cells screened by the screening method.

The present disclosure is also directed to providing a pharmaceutical composition for preventing or treating a neurological disease, which contains the neuronal regeneration-promoting cells as an active ingredient.

Other purposes and advantages of the present disclosure will become more apparent by the following detailed description, claims and drawings.

### [Technical Solution]

In an aspect, the present disclosure provides a method for screening mesenchymal stem cell-derived, neuronal regeneration-promoting cells having neuronal regeneration activity, which includes:
i) a step of preparing cells differentiated from mesenchymal stem cells; and
ii) a step of screening cells in which one or more marker selected from a group consisting of CD121a, CD106 and CD112 is up-regulated from among the differentiated cells of the step i) as compared to mesenchymal stem cells before differentiation.

In another aspect, the present disclosure provides a method for screening mesenchymal stem cell-derived, neuronal regeneration-promoting cells having neuronal regeneration activity, which includes:
i) a step of preparing cells differentiated from mesenchymal stem cells; and
ii) a step of screening cells in which one or more marker selected from a group consisting of CD26 and CD141 is down-regulated from among the differentiated cells of the step i) as compared to mesenchymal stem cells before differentiation.

The inventors of the present disclosure have differentiated mesenchymal stem cells derived from various sources and investigated the expression pattern of various markers in the differentiated various cells. As a result, it was surprisingly confirmed that the cells differentiated into neuronal regeneration-promoting cells show common tendency in the expression pattern of specific markers (e.g., CD markers such as CD121a, CD106 and CD112).

In the present disclosure, the term "neuronal regeneration-promoting cell", or "NRPC" refers to a cell differentiated from a mesenchymal stem cell, which has neuronal regeneration effect (e.g., an effect of promoting neuronal regeneration directly or indirectly in terms of structure or function by myelinating damaged peripheral nerves or secreting cytokines necessary for neuronal regeneration).

According to a specific exemplary embodiment of the present disclosure, the screening method includes:
i) a step of preparing cells differentiated from mesenchymal stem cells;
ii) a step of screening cells in which one or more marker selected from a group consisting of CD121a, CD106 and CD112 is up-regulated from among the differentiated cells of the step i) as compared to mesenchymal stem cells before differentiation; and
iii) a step of screening cells in which one or more marker selected from a group consisting of CD26 and CD141 is down-regulated from among the differentiated cells of the step i) as compared to mesenchymal stem cells before differentiation.

According to a specific exemplary embodiment of the present disclosure, the screening method includes:
i) a step of preparing cells differentiated from mesenchymal stem cells;
ii) a step of screening cells in which one or more marker selected from a group consisting of CD26 and CD141 is down-regulated from among the differentiated cells of the step i) as compared to mesenchymal stem cells before differentiation; and
iii) a step of screening cells in which one or more marker selected from a group consisting of CD121a, CD106 and CD112 is up-regulated from among the differentiated cells of the step i) as compared to mesenchymal stem cells before differentiation.

In the present disclosure, the term "stem cell" refers to a cell which can replicate itself and at the same time has the ability to differentiate into two or more types of cells. The stem cells include adult stem cells, pluripotent stem cells, induced pluripotent stem cells or embryonic stem cells. Specifically, they may be mesenchymal stem cells.

In the present disclosure, the term "mesenchymal stem cell" refers to an undifferentiated stem cell isolated from the tissue of human or a mammal. The mesenchymal stem cell may be derived from various tissues. Especially, it may be derived from one or more selected from a group consisting of the tonsil, umbilical cord, umbilical cord blood, bone marrow, fat, muscle, nerve, skin, amnion, chorion, decidua and placenta. The techniques for isolating stem cells from each tissue are well known in the art.

According to a specific exemplary embodiment of the present disclosure, the mesenchymal stem cell is derived from the tonsil or fat.

In an example of the present disclosure, it was verified that it is the most preferred to use mesenchymal stem cells derived from the tonsil or fat.

In the present disclosure, the term "CD" or "cluster of differentiation" refers to a surface molecular structure present on cell surface. Since some cell populations share the same CD molecules, they are used to distinguish cell populations (i.e., as markers). Cells of the same lineage have the same CD molecules but even the same cell population have different CD molecules depending on the stage of differentiation or activation. Therefore, they are usefully used to identify the lineage, differentiation, activation, etc. of cells.

In an example of the present disclosure, it was verified from the comparison of the expression pattern of CD molecules in the stem cell-derived, neuronal regeneration-promoting cells having neuronal regeneration activity of the present disclosure and mesenchymal stem cells that the neuronal regeneration-promoting cells according to the present disclosure are different from the mesenchymal stem cells. In the present disclosure, CD10, CD39, CD106, CD112, CD121a, CD338, etc. the expression of which is up-regulated as compared to the mesenchymal stem cells or CD26 CD54, CD126, CD141, etc. the expression of which is down-regulated may be used as differentiation markers of neuronal regeneration-promoting cells.

According to a specific exemplary embodiment of the present disclosure, the differentiated cells of the step i) are differentiated from neurospheres formed by culturing mesenchymal stem cells.

According to a specific exemplary embodiment of the present disclosure, the neuronal regeneration activity includes the myelination of peripheral nerves.

In the present disclosure, the term "myelination" refers to the phenomenon where myelin surrounds the axons of peripheral nerves to increase the rate at which stimulus is delivered. Damaged peripheral nerves are normalized (i.e., regenerated) through myelination.

In an example of the present disclosure, some of the candidate cells screened by the screening method of the present disclosure were myelinated cytomorphologically through co-culturing with dorsal root ganglia.

In another aspect, the present disclosure provides neuronal regeneration-promoting cells screened by the screening method described above.

According to a specific exemplary embodiment of the present disclosure, the neuronal regeneration-promoting cells have the following characteristics:
a) the expression of the markers CD121a, CD106 and CD112 is up-regulated as compared to mesenchymal stem cells before differentiation; and
b) the expression of the markers CD26 and CD141 is down-regulated as compared to mesenchymal stem cells before differentiation.

In the neuronal regeneration-promoting cells, the expression of the marker CD121a is up-regulated by specifically 30% or more, more specifically 40% or more, as compared to mesenchymal stem cells before differentiation.

In an example of the present disclosure, it was verified that the expression of the marker CD121a is up-regulated by 94% in T-MSC-1-1-derived neuronal regeneration-promoting cells, 71% in T-MSC-1-2-derived neuronal regeneration-promoting cells, 51% in T-MSC-1-3-derived neuronal regeneration-promoting cells and 48% in T-MSC-1-4-derived neuronal regeneration-promoting cells, 66% or more on average, as compared to mesenchymal stem cells before differentiation.

In the neuronal regeneration-promoting cells, the expression of the marker CD106 is up-regulated by specifically 5% or more, more specifically 10% or more, as compared to mesenchymal stem cells before differentiation.

In an example of the present disclosure, it was verified that the expression of the marker CD106 is up-regulated by 30% in T-MSC-1-1-derived neuronal regeneration-promoting cells, 11% in T-MSC-1-2-derived neuronal regeneration-promoting cells, 16% in T-MSC-1-3-derived neuronal regeneration-promoting cells and 13% in T-MSC-1-4-derived neuronal regeneration-promoting cells, 17% or more on average, as compared to mesenchymal stem cells before differentiation.

In the neuronal regeneration-promoting cells, the expression of the marker CD112 is up-regulated by specifically 10% or more, more specifically 15% or more, as compared to mesenchymal stem cells before differentiation.

In an example of the present disclosure, it was verified that the expression of the marker CD112 is up-regulated by 49% in T-MSC-1-1-derived neuronal regeneration-promoting cells, 25% in T-MSC-1-2-derived neuronal regeneration-promoting cells, 30% in T-MSC-1-3-derived neuronal regeneration-promoting cells and 19% in T-MSC-1-4-derived neuronal regeneration-promoting cells, 30% or more on average, as compared to mesenchymal stem cells before differentiation.

According to a specific exemplary embodiment of the present disclosure, in the neuronal regeneration-promoting cells, the expression of the marker CD26 is down-regulated as compared to mesenchymal stem cells before differentiation.

In the neuronal regeneration-promoting cells, the expression of the marker CD26 is down-regulated by specifically 5% or more, more specifically 8% or more, as compared to mesenchymal stem cells before differentiation.

In an example of the present disclosure, the expression of the marker CD26 is down-regulated by 9% in T-MSC-1-1-derived neuronal regeneration-promoting cells, 11% in T-MSC-1-2-derived neuronal regeneration-promoting cells, 27% in T-MSC-1-3-derived neuronal regeneration-promoting cells and 16% in T-MSC-1-4-derived neuronal regeneration-promoting cells, 16% or more on average, as compared to mesenchymal stem cells before differentiation.

In the neuronal regeneration-promoting cells, the expression of the marker CD141 is down-regulated by specifically 5% or more, more specifically 8% or more, as compared to mesenchymal stem cells before differentiation.

In an example of the present disclosure, the expression of the marker CD141 is down-regulated by 9% in T-MSC-1-1-derived neuronal regeneration-promoting cells, 20% in T-MSC-1-2-derived neuronal regeneration-promoting cells, 16% in T-MSC-1-3-derived neuronal regeneration-promoting cells and 38% in T-MSC-1-4-derived neuronal regeneration-promoting cells, 20% or more on average, as compared to mesenchymal stem cells before differentiation.

In another aspect, the present disclosure provides a pharmaceutical composition for preventing or treating a neurological disease, which contains the neuronal regeneration-promoting cells as an active ingredient.

In another aspect, the present disclosure provides a method for treating a neurological disease, which includes a step of administering an effective amount of the neuronal regeneration-promoting cells to a subject.

In another aspect, the present disclosure provides a use of the neuronal regeneration-promoting cells in therapy.

In the present disclosure, the term "neurological disease" refers to a disease caused by damage to neural tissues due to intrinsic factors such as heredity, aging, etc. or extrinsic factors such as trauma, etc.

According to a specific exemplary embodiment of the present disclosure, the neurological disease is one or more disease selected from a group consisting of Charcot-Marie-Tooth neuropathy, diabetic peripheral neuropathy, spinal cord injury, amyotrophic lateral sclerosis, carpal tunnel syndrome, infantile paralysis, leprosy, muscular dystrophy, polymyositis and myasthenia gravis.

In the present disclosure, the term "subject" refers to an individual requiring the administration of the composition or the neuronal regeneration-promoting cells of the present disclosure, and includes mammals, birds, reptiles, amphibians, fish, etc. without limitation.

In the present disclosure, "prevention" refers to any action of inhibiting or delaying a neurological disease by administering the composition according to the present disclosure. And, "treatment" refers to any action of improving or favorably changing the symptoms of a neurological disease by administering the composition according to the present disclosure.

According to a specific exemplary embodiment of the present disclosure, the pharmaceutical composition of the present disclosure contains a pharmaceutically acceptable carrier or excipient.

The pharmaceutical composition of the present disclosure may be prepared into a single-dose or multi-dose formulation using a pharmaceutically acceptable carrier and/or excipient according to a method that can be easily carried out by those having ordinary knowledge in the art to which the present disclosure belongs.

The pharmaceutical composition according to the present disclosure may be prepared into various formulations according to common methods. For example, it may be prepared into an oral formulation such as a powder, a granule, a tablet, a capsule, a suspension, an emulsion, a syrup, etc. and may also be prepared into a formulation for external application, a suppository or a sterilized injection solution.

The composition of the present disclosure may contain one or more known active ingredient having an effect of preventing or treating a neurological disease together with the stem cell-derived, neuronal regeneration-promoting cells having neuronal regeneration activity.

The pharmaceutical composition of the present disclosure may be administered orally or parenterally. Specifically, it may be administered or parenterally, e.g., by intravenous injection, transdermal administration, subcutaneous injection, intramuscular injection, intravitreal injection, subretinal injection, suprachoroidal injection, eye drop administration, intracerebroventricular injection, intrathecal injection, intraamniotic injection, intraarterial injection, intraarticular injection, intracardiac injection, intracavernous injection, intracerebral injection, intracisternal injection, intracoronary injection, intracranial injection, intradural injection, epidural injection, intrahippocampal injection, intranasal injection, intraosseous injection, intraperitoneal injection, intrapleural injection, intraspinal injection, intrathoracic injection, intrathymic injection, intrauterine injection, intravaginal injection, intraventricular injection, intravesical injection, subconjunctival injection, intratumoral injection, topical injection, etc.

Formulations for the parenteral administration include a sterilized aqueous solution, a nonaqueous solution, a suspension, an emulsion, a freeze-dried formulation and a suppository. For the nonaqueous solution or suspension, propylene glycol, polyethylene glycol, a vegetable oil such as olive oil, an injectable ester such as ethyl oleate, etc. may be used. As a base of the suppository, witepsol, macrogol, Tween 61, cocoa butter, laurin butter, glycerogelatin, etc. may be used.

The administration dosage of the pharmaceutical composition of the present disclosure may vary depending on various factors such as formulation method, administration method, administration time, administration route, the response to be achieved with the administration of the pharmaceutical composition and the extent thereof, the age, body weight, general health condition, pathological condition or severity, sex, diet and excretion rate of a subject to which the pharmaceutical composition is administered and other drugs or ingredients used together and similar factors well known in the medical field, and an administration dosage effective for the desired treatment may be easily determined and prescribed by those having ordinary knowledge in the art.

The administration route and administration method of the pharmaceutical composition of the present disclosure may be independent from each other and are not specially limited as long as the pharmaceutical composition can reach the target site.

### [Advantageous Effects]

The features and advantages of the present disclosure may be summarized as follows:
(i) The present disclosure provides a method for screening mesenchymal stem cell-derived, neuronal regeneration-promoting cells having neuronal regeneration activity and a pharmaceutical composition containing the neuronal regeneration-promoting cells.
(ii) The neuronal regeneration-promoting cells of the present disclosure are completely different from stem cells in terms of the expression pattern of a CD marker and exhibit an excellent neuronal regeneration effect. Accordingly, they can be applied in various fields for preventing or treating neurological diseases.

### [Brief Description of Drawings]

FIG. 1 shows the images of neuronal regeneration-promoting cells induced from the tonsil-derived T-MSC-1-1 mesenchymal stem cells.
FIG. 2 shows heat maps visualizing the expression of CD markers in the neuronal regeneration-promoting cells according to the present disclosure through CD marker screening.
FIGS. 3a and 3b show a result of screening CD markers showing difference in the expression level of neuronal regeneration-promoting cells as compared to tonsil-derived mesenchymal stem cells. FIG. 3a shows a result of comparing the CD markers the expression level of which has increased as compared to tonsil-derived mesenchymal stem cells, and FIG. 3b shows a result of comparing the CD markers the expression level of which has decreased as compared to tonsil-derived mesenchymal stem cells.
FIG. 4 shows a result of comparing the expression pattern of the CD markers the expression of which has increased and the CD markers the expression of which has decreased in neuronal regeneration-promoting cells as compared to tonsil-derived mesenchymal stem cells.
FIG. 5 shows a result of screening the CD markers the expression of which has increased or decreased in neuronal regeneration-promoting cells.
FIG. 6 shows a result of comparing the expression pattern of the markers CD121a, CD106 and CD112 the expression of which has increased in neuronal regeneration-promoting cells as compared to tonsil-derived mesenchymal stem cells.
FIG. 7 shows a result of comparing the expression pattern of the markers CD26 and CD141 the expression of which has decreased in neuronal regeneration-promoting cells as compared to tonsil-derived mesenchymal stem cells.
FIG. 8 shows a result of comparing the expression pattern of CD markers in tonsil-derived mesenchymal stem cells (T-MSCs) and neuronal regeneration-promoting cells (NRPCs) using heat maps.
FIG. 9 shows a result of conducting neurite outgrowth assay to compare the growth of neurites from neuronal regeneration-promoting cells.
FIG. 10 shows that myelination was achieved cytomorphologically in some of the candidate cells co-cultured with dorsal root ganglia.
FIG. 11 shows a result of conducting flow cytometry for up-regulated and down-regulated CD markers screened by CD screening using individual antibodies.
FIG. 12 shows a result of visualizing the cytokine array assay of T-MSCs and NRPCs using heat maps (left) and the proportion of the cytokines increased in NRPCs as compared to the T-MSCs (right) (fold change: NRPC 1-1/T-MSC 1-1, NRPC 1-2/ T-MSC 1-2).

### [Best Mode]

Hereinafter, the present disclosure will be described in more detail through examples. The examples are provided only to describe the present disclosure more specifically and it will be obvious to those having ordinary knowledge in the art that the scope of the present disclosure is not limited by the examples.

### Examples

### Example 1. Preparation of mesenchymal stem cells

### 1-1. Isolation and culturing of tonsil-derived mesenchymal stem cells

Tonsil tissues derived from many donors acquired from Ewha Womans University College of Medicine were put in a tube holding 10 mL of DPBS (Dulbecco's phosphate-buffered saline) supplemented with 20 µg/mL gentamicin, centrifuged at 1,500 rpm for 5 minutes and then washed twice. The washed tonsil issues were sliced using sterilized scissors.

In order to isolate tonsil-derived mesenchymal stem cells from the tonsil issues, after adding an enzymatic reaction solution of the same weight, the tonsil issues were incubated in a shaking incubator at 37 °C and 200 rpm for 60 minutes. The composition of the enzymatic reaction solution is described in Table 1.

**[Table 1]**

| Final concentration | Ingredients |
|---|---|
| 2 mg/mL | Trypsin |
| 1.2 U/mL | Dispase |
| 1 mg/mL | Type 1 collagenase |
| 20 µg/mL | DNase 1 |
| - | HBSS (Hank's balanced salt solution) |

After adding 5% FBS (fetal bovine serum) to the culture, the mixture was centrifuged at 1,500 rpm for 5 minutes. After the centrifugation, the supernatant was removed and the remaining pellets were resuspended in 30 mL of DPBS and then centrifuged at 1,500 rpm for 5 minutes. After the centrifugation, the supernatant was removed and the remaining pellets were resuspended in 10 mL of DPBS to prepare a suspension. The suspension was passed through a 100-µm filter. The tonsil-derived mesenchymal stem cells remaining in the filter were washed with 20 mL of DPBS and then centrifuged at 1,500 rpm for 5 minutes. After the centrifugation, the supernatant was removed and incubation was performed in a constant-temperature water bath at 37 °C for 5 minutes after adding an ACK lysis buffer. After adding DPBS to the suspension, centrifugation was conducted at 1,500 rpm for 5 minutes. After the centrifugation, the supernatant was removed and the remaining pellets were resuspended in high-glucose DMEM (10% FBS, 20 µg/mL gentamicin) to prepare a cell suspension. Then, the number of cells in the prepared cell suspension was counted. The cell suspension was seeded in a T175 flask and incubated at 37°C in a 5% CO₂ incubator.

### 1-2. Isolation and culturing of adipose-derived mesenchymal stem cells

Adipose-derived mesenchymal stem cells were purchased from Lonza (human adipose-derived stem cells, Cat#PT-5006, Lonza, Switzerland). The adipose-derived mesenchymal stem cells were cultured using a medium provided by Lonza (Bulletkit ADSD, Cat#PT-4505).

### Example 2. Formation of neurospheres

Neurospheres were formed by culturing the mesenchymal stem cells of Example 1. Specifically, the mesenchymal stem cells were subcultured to 4-7 passages. After removing the culture medium, the mesenchymal stem cells were washed with DPBS. After treating the washed cells with TrypLE, the harvested cells were counted. After centrifuging the harvested cells and removing the supernatant, they were resuspended in a neurosphere formation medium. The composition of the neurosphere formation medium is described in Table 2.

**[Table 2]**

| Final concentration | Ingredients |
|---|---|
| - | DMEM/F12 with GlutaMAX |
| 20 ng/mL | Basic fibroblast growth factor |
| 20 ng/mL | Epidermal growth factor |
| 1x | B27 supplement |
| 20 µg/mL | Gentamicin |

The cells resuspended in the neurosphere (1x10⁶ cells) formation medium were seeded on an ultra-low attachment dish (60 mm). The seeded cells were cultured for 3 days under the condition of 37 °C and 5% CO₂. After the culturing for 3 days, the neurospheres formed on the dish were collected in a 15-mL tube. After centrifuging the collected cells and removing the supernatant, a neurosphere suspension was prepared by adding a fresh neurosphere formation medium. The neurosphere suspension was transferred to an ultra-low attachment dish and the neurospheres were cultured for 4 days under the condition of 37 °C and 5% CO₂.

### Example 3. Differentiation into candidate cells of neuronal regeneration-promoting cells (NRPCs) using neurospheres

The neurospheres formed in Example 2 were crushed finely using a 23-26G syringe needle. The crushed neurospheres were transferred to a 15-mL tube using a pipette and then centrifuged. After removing the supernatant, the crushed neurospheres were resuspended by adding a neuronal regeneration-promoting cell induction medium to the tube. Various neuronal regeneration-promoting cell induction media were prepared by combining three or more of 1) 5-20% FBS (fetal bovine serum), 2) 5-20 ng/mL bFGF (Peprotech, USA), 3) 100-400 µM butylated hydroxyanisole (Sigma, USA), 4) 5-40 µM forskolin (MedCheExpress, USA), 5) 0.1-10% N2 supplement (GIBCO, USA), 6) 1-100 ng/mL brain-derived neurotrophic factor (BDNF, Sigma-Aldrich, USA), 7) 1-100 ng/mL nerve growth factor (NGF, Santa Cruz, USA), 8) 0.01-1 ng/mL sonic hedgehog (SHH, R & D Systems, USA), 9) 1-10 ng/mL PDGF-AA (platelet-derived growth factor-AA, Peprotech, USA) and 10) 50-300 ng/mL heregulin-beta1, Peprotech, USA) in DMEM/F12 containing GlutaMAX.

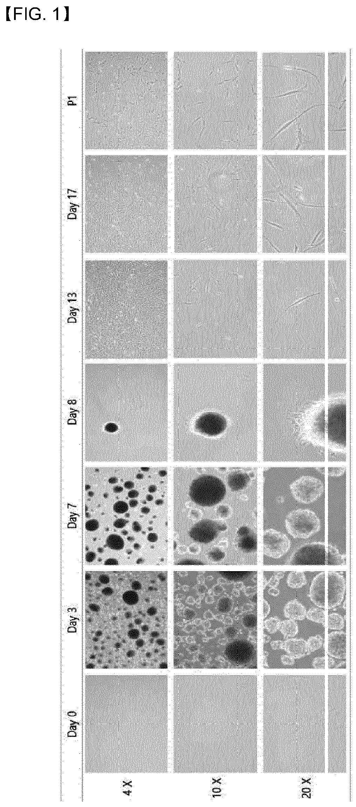

The neurospheres resuspended in the various media were seeded onto a T175 flask coated with laminin (2 µg/mL). The seeded neurospheres were cultured for 8-10 days while exchanging the neuronal regeneration-promoting cell induction medium at 3-day intervals (FIG. 1).

### Example 4. First screening of candidate cells of neuronal regeneration-promoting cells through confirmation of myelination of peripheral nerves

It was investigated whether the neuronal regeneration-promoting cell candidates prepared in Example 3 have the ability of myelinating peripheral nerves. Specifically, the differentiated neuronal regeneration-promoting cell candidates were co-cultured with dorsal root ganglia (DRG) and it was investigated whether myelination occurred.

Dorsal root ganglion (DRG) cells isolated from rats were purchased from Lonza (rat dorsal root ganglion cells, Cat# R-DRG-505, Lonza, Switzerland). The candidate cells were co-cultured with the purchased dorsal root ganglia. The DRG cells were cultured using a culture medium provided by Lonza (primary neuron growth medium bullet kit (PNGM), Cat# CC-4461).

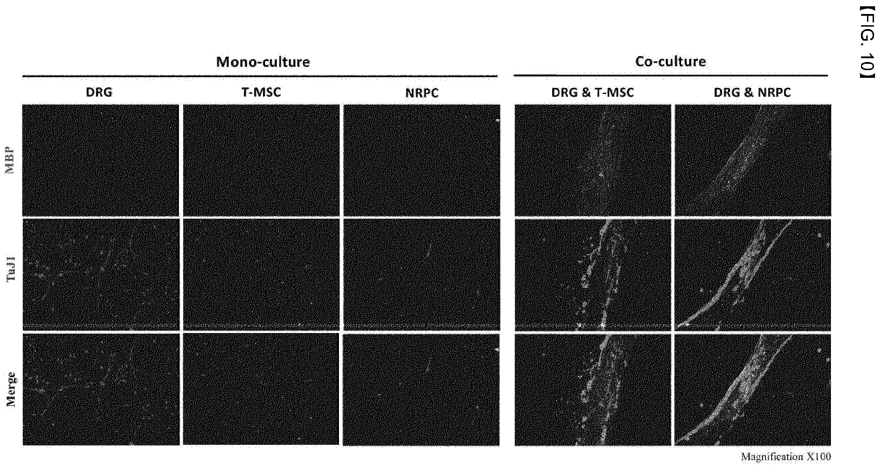

The culture medium was exchanged every 3 days. As a result of co-culturing the candidate cells with the dorsal root ganglia, it was confirmed that myelination was achieved cytomorphologically in some of the cells (FIG. 10).

### Example 5. Second screening of neuronal regeneration-promoting cells through analysis of CD marker expression

The expression of a total of 242 CD markers was analyzed in T-MSC-1-1 (tonsil-derived mesenchymal stem cells 1), T-MSC-1-2 (tonsil-derived mesenchymal stem cells 2), T-MSC-1-3 (tonsil-derived mesenchymal stem cells 3) and T-MSC-1-4 (tonsil-derived mesenchymal stem cells 4), wherein myelination was confirmed cytomorphologically among the neuronal regeneration-promoting cell candidates in Example 4, and neuronal regeneration-promoting cells differentiated therefrom.

For the analysis of CD markers, 3x10⁷ target cells were collected. The target cell were washed with DPBS and then centrifuged at 2000 rpm for 5 minutes. After removing the supernatant and washing once with DPBS, centrifugation was conducted and the remaining pellets were resuspended in 30 mL of a FACS buffer. 100 µL of the cell suspension (1x10⁵ cells) was seeded in each well of a round-bottomed 96-well plate. Then, 10 µL of primary antibodies of CD markers were added to each well of the 96-well plate. After reaction for 30 minutes on ice with the light blocked, each well was washed with 100 µL of a FACS buffer and then centrifugation was performed at 300 g for 5 minutes. After removing the supernatant and adding 200 µL of a FACS buffer to each well, centrifugation was performed at 300 g for 5 minutes. Secondary antibodies were prepared in a FACS buffer at a ratio of 1:200 (1.25 µg/mL). After the centrifugation was completed, the supernatant was removed and then 100 µL of the prepared secondary antibodies were added to each well. After reaction for 20-30 minutes on ice with the light blocked, each well was washed with 100 µL of a FACS buffer and then centrifugation was performed at 300 g for 5 minutes. After removing the supernatant, the target cells were washed by adding 200 µL of a FACS buffer to each well. The washing procedure was repeated twice. After the washing, the cells were resuspended by adding 200 µL of a FACS buffer to each well and the expression of CD markers in the target cells was investigated by flow cytometry or FACS (fluorescence-activated cell sorting).

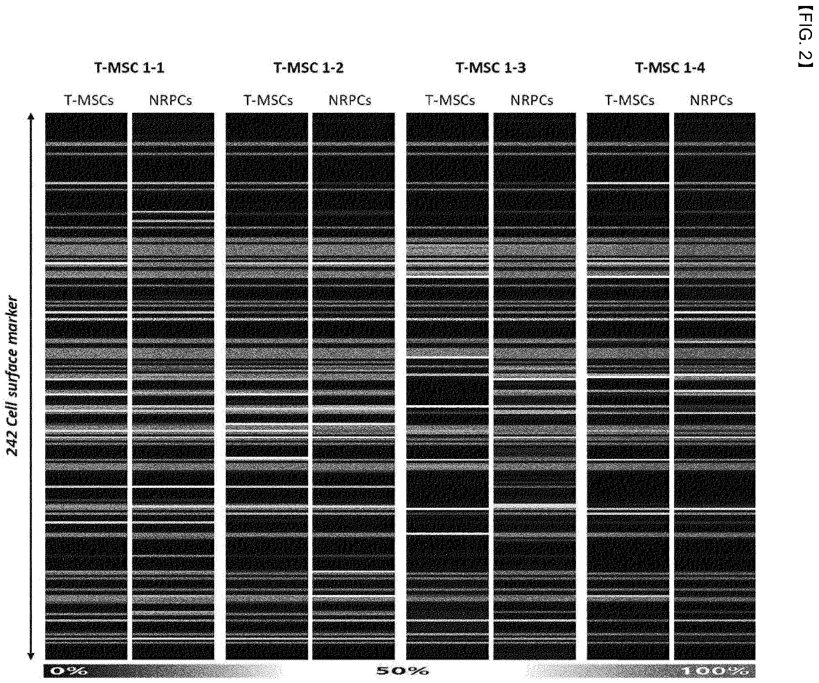

The result of comparing the expression of CD markers in the induced neuronal regeneration-promoting cells using heat maps is shown in FIG. 2. As shown in FIG. 2, the neuronal regeneration-promoting cells (NRPCs) and the mesenchymal stem cells (MSCs) showed similar CD marker expression patterns but showed difference in the expression pattern of some markers.

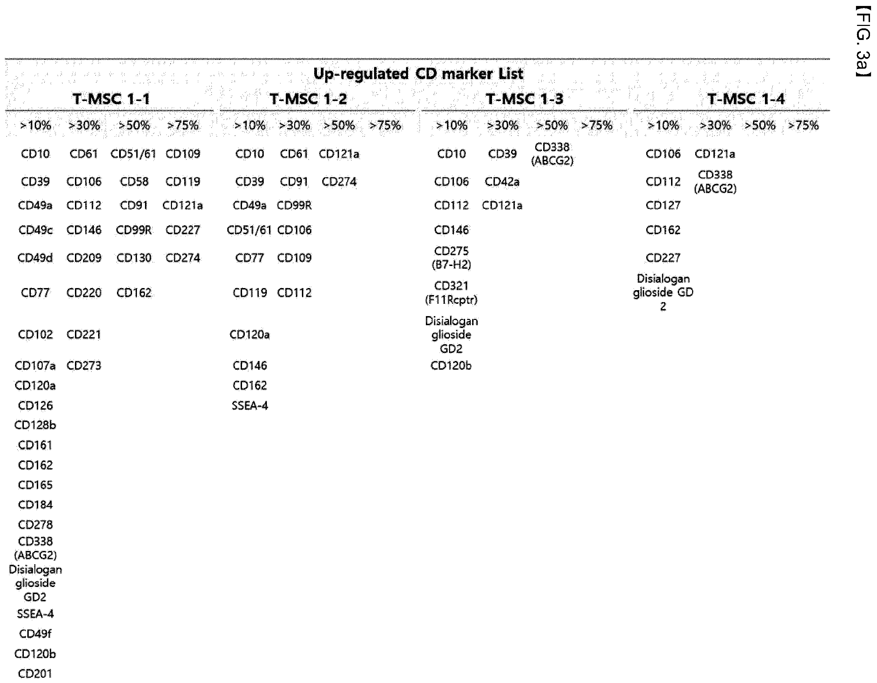

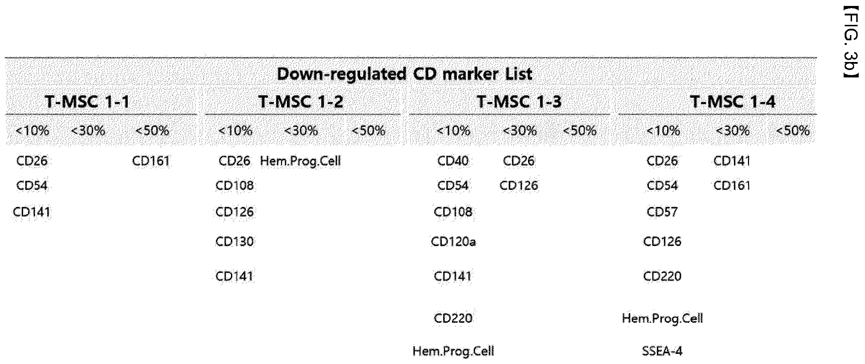

The CD marker expression pattern of the T-MSC-1-1, T-MSC-1-2, T-MSC-1-3 and T-MSC-1-4 mesenchymal stem cells (MSCs) and the neuronal regeneration-promoting cells (NRPCs) was compared to select the CD markers the expression of which has increased or decreased as differentiation markers of neuronal regeneration-promoting cells. The CD markers the expression of which has increased or decreased are shown in FIG. 3.

As shown in FIG. 3a, the CD markers the expression of which has increased in the neuronal regeneration-promoting cells derived from T-MSC-1-1, T-MSC-1-2, T-MSC-1-3 and T-MSC-1-4 (in at least three of the four NRPCs) as compared to the tonsil-derived mesenchymal stem cells were CD10, CD39, CD106, CD112, CD121a, CD338, etc. And, as shown in FIG. 3b, the CD markers the expression of which has decreased (in at least three of the four NRPCs) were CD26 CD54, CD126, CD141, etc.

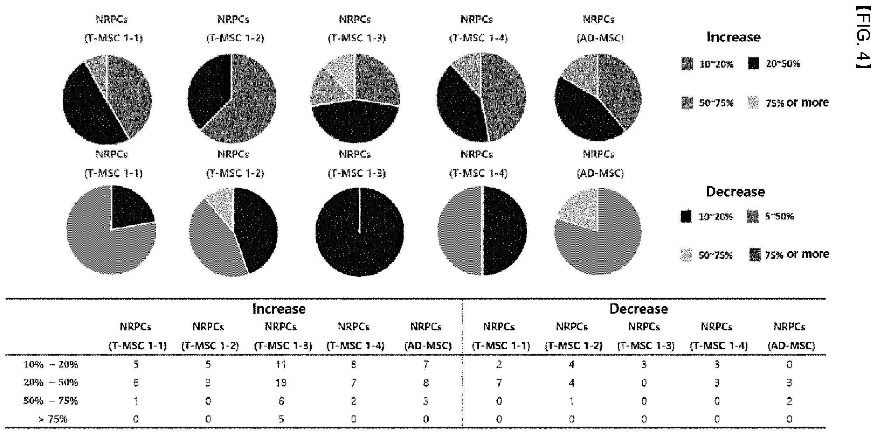

The result of comparing the increase and decrease of the CD markers in the tonsil-derived neuronal regeneration-promoting cells is shown in FIG. 4.

As shown in FIG. 4, the expression of 12 CD markers was increased and the expression of 9 CD markers was decreased in the neuronal regeneration-promoting cells derived from T-MSC-1-1. And, the expression of 8 CD markers was increased and the expression of 9 CD markers was decreased in the neuronal regeneration-promoting cells derived from T-MSC-1-2. The expression of 40 CD markers was increased and the expression of 3 CD markers was decreased in the neuronal regeneration-promoting cells derived from T-MSC-1-3. The expression of 17 CD markers was increased and the expression of 6 CD markers was decreased in the neuronal regeneration-promoting cells derived from T-MSC-1-4.

From the above results, the CD markers the expression of which has increased or decreased commonly in the neuronal regeneration-promoting cells derived from T-MSC-1-1, T-MSC-1-2, T-MSC-1-3 and T-MSC-1-4 were screened. The screened markers are as follows:
- The CD markers the expression of which has increased commonly: CD106, CD112 and CD121a.
- The CD markers the expression of which has decreased commonly: CD26 and CD141.

The pattern of the CD markers the expression of which has increased or decreased commonly was observed identically also in the neuronal regeneration-promoting cells differentiated from the adipose-derived mesenchymal stem cells of Example 1-2.

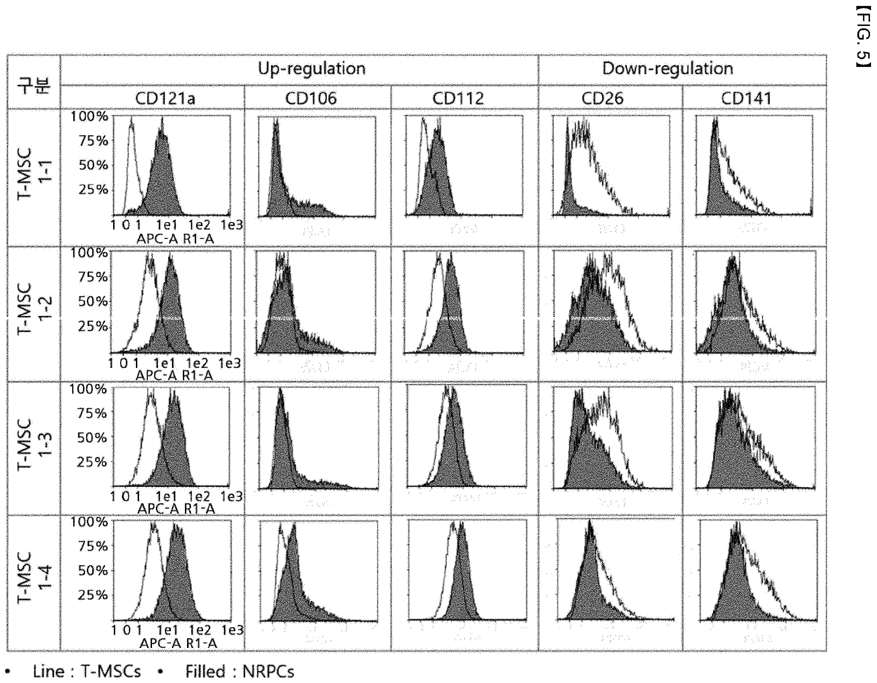

The CD screening result of the CD markers the expression of which has increased or decreased in the neuronal regeneration-promoting cells derived from T-MSC-1-1, T-MSC-1-2, T-MSC-1-3 and T-MSC-1-4 is shown in FIG. 5.

As shown in FIG. 5, the expression level of the CD markers the expression of which has increased commonly in the neuronal regeneration-promoting cells, i.e., CD121a, CD106 and CD112, has increased by 10% or more after the differentiation. Meanwhile, the expression level of the markers the expression of which has decreased commonly, i.e., CD26 and CD141, had decreased by about 9% or more. This result suggests that the markers CD121a, CD106, CD112, CD26 and CD141 expression of which has changed commonly can be used as differentiation markers of neuronal regeneration-promoting cells. Especially, CD121a, CD106 and CD112 can be used as representative differentiation markers.

### 6-1. Comparison of expression of co-expression markers CD121a, CD106 and CD112

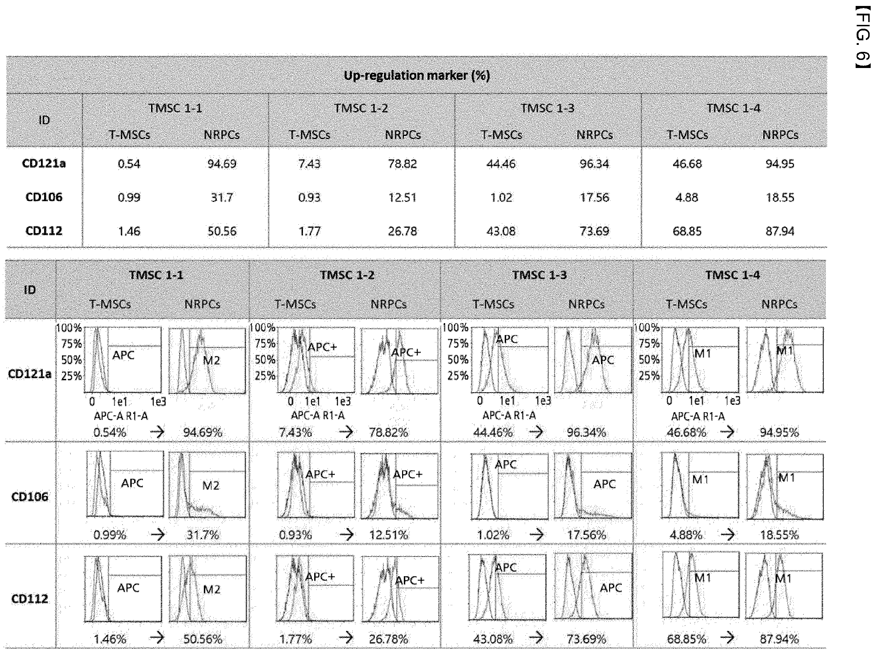

The expression of the markers CD121a, CD106 and CD112 has increased commonly in the neuronal regeneration-promoting cells derived from T-MSC-1-1, T-MSC-1-2, T-MSC-1-3 and T-MSC-1-4 by 10% or more as compared to the mesenchymal stem cells, which is the most prominent feature of the neuronal regeneration-promoting cells. The result of comparing the expression of the markers CD121a, CD106 and CD112 in the neuronal regeneration-promoting cells derived from T-MSC-1-1, T-MSC-1-2, T-MSC-1-3 and T-MSC-1-4 is shown in FIG. 6.

As shown in FIG. 6, the expression of the markers CD121a, CD106 and CD112 was increased remarkably in the neuronal regeneration-promoting cells (NRPCs) as compared to the tonsil-derived mesenchymal stem cells (T-MSCs).

### 6-2. Comparison of expression of co-expression markers CD26 and CD141

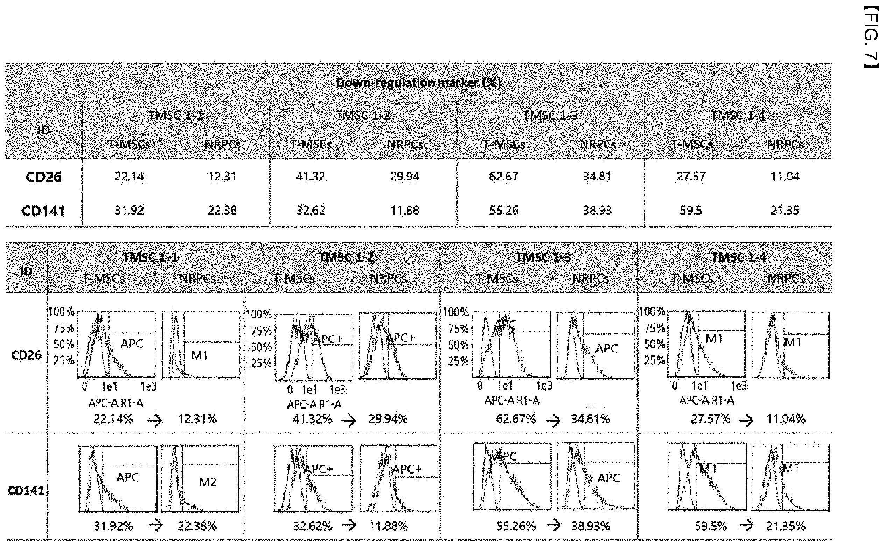

The expression of the markers CD26 and CD141 had decreased commonly in the neuronal regeneration-promoting cells derived from T-MSC-1-1, T-MSC-1-2, T-MSC-1-3 and T-MSC-1-4. The result of comparing the expression of the CD markers in the neuronal regeneration-promoting cells derived from T-MSC-1-1, T-MSC-1-2, T-MSC-1-3 and T-MSC-1-4 is shown in FIG. 7.

As shown in FIG. 7, the expression of CD26 and CD141 was decreased in the neuronal regeneration-promoting cells as compared to the tonsil-derived mesenchymal stem cells.

### 6-3. Comparison of expression pattern of CD markers

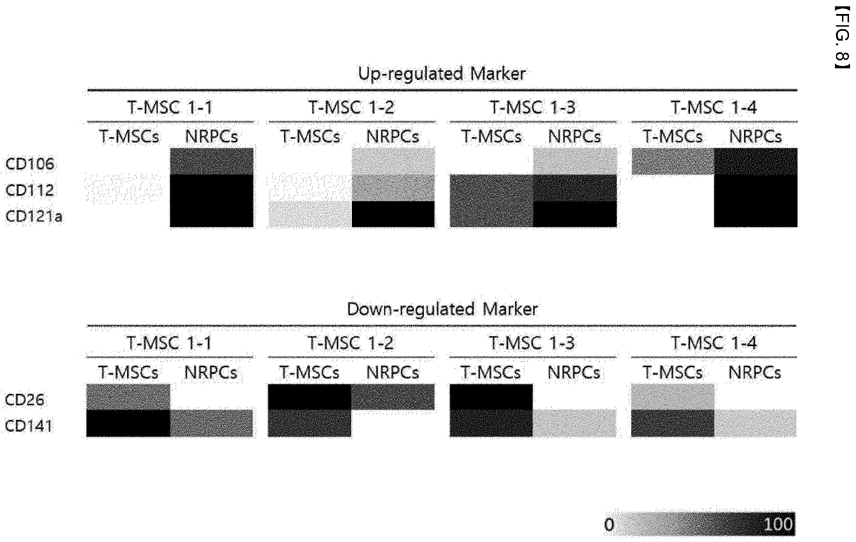

In order to compare the CD marker expression pattern in the tonsil-derived mesenchymal stem cells and the neuronal regeneration-promoting cells, heat maps were constructed based on the result of comparing the expression of the co-expression CD markers in Examples 6-1 and 6-2. The result is shown in FIG. 8.

As shown in FIG. 8, the neuronal regeneration-promoting cells showed different expression of the co-expression markers from the tonsil-derived mesenchymal stem cells.

### 6-4. Average expression of co-expression CD markers

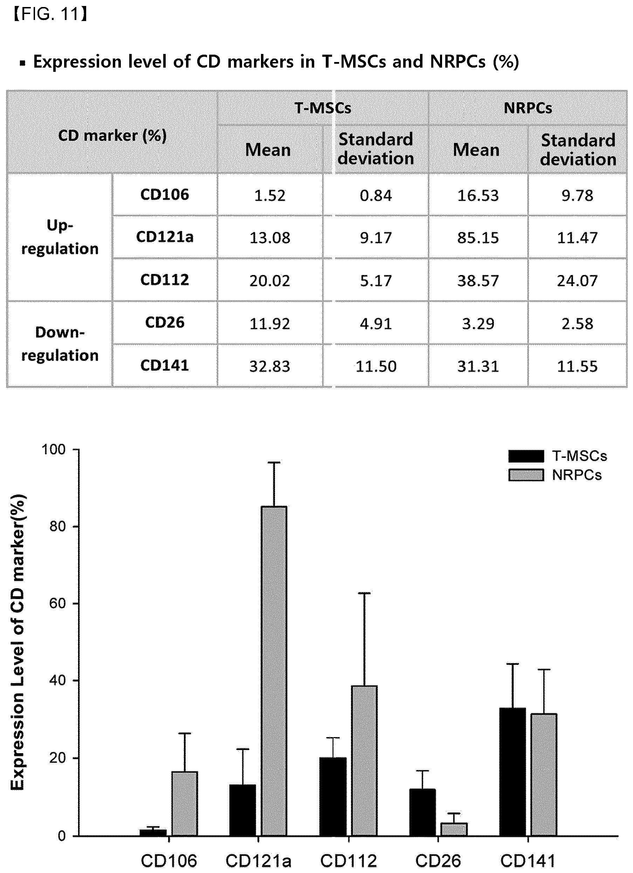

In order to investigate whether the expression pattern of the CD markers in the tonsil-derived mesenchymal stem cells and the neuronal regeneration-promoting cells is maintained after freezing of the cells, the expression of the co-expression CD markers in live cells, frozen cells and thawed cells was confirmed. The result is shown in FIG. 11.

As shown in FIG. 11, the expression of CD106, CD121a and CD112 was increased and the expression of CD26 and CD141 was decreased in the neuronal regeneration-promoting cells as compared to the tonsil-derived mesenchymal stem cell even after freezing. The neuronal regeneration-promoting cells showed different expression of the co-expression markers from the tonsil-derived mesenchymal stem cells regardless of freezing.

### Example 7. Neurite outgrowth effect of neuronal regeneration-promoting cells

The neurite (or neuronal process), which projects from the cell body of a neuron, is known to be involved in the transport of the substances necessary for growth and regeneration of axons, neurotransmitters, nerve growth factors, etc. (L McKerracher et al., Spinal Cord Repair: Strategies to Promote Axon Regeneration, Neurobiol Dis, 2001). Neurite outgrowth assay was conducted to compare neurite growth in the neuronal regeneration-promoting cells of the present disclosure.

N1E-115 cells (mouse neuroblastoma cells, ATCC, USA) were cultured and seeded on a microporous filter (neurite outgrowth assay kit, Millipore, USA). The seeded cells were cultured for 48 hours in a culture medium from which the neuronal regeneration-promoting cells or the stem cells were collected. Absorbance was measured after staining the neurites projected through a fine porous filter. It was confirmed from the neurite outgrowth assay that the culture of the neuronal regeneration-promoting cells regulate or stimulate the growth of neurites (axons) in the N1E-115 (mouse neuroblastoma) cells.

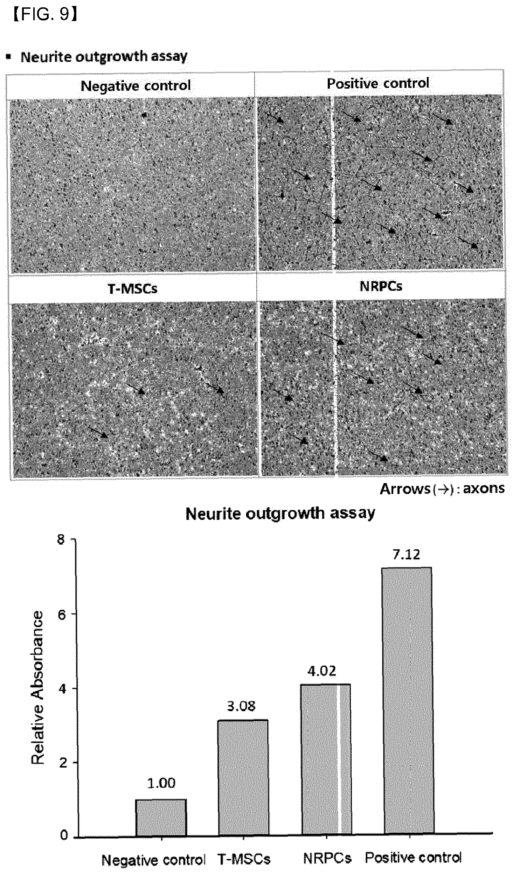

The growth of neurites in the N1E-115 cells cultured with the neuronal regeneration-promoting cells derived from T-MSC-1-2 was compared. The result is shown in FIG. 9 (NRPCs: neuronal regeneration-promoting cells derived from T-MSC-1-2, T-MSCs: T-MSC-1-2, Negative control: negative control group, Positive control: positive control group). A large number of neurites was observed in the neuronal regeneration-promoting cells as compared to the tonsil-derived stem cells, and absorbance was also increased in the neuronal regeneration-promoting cells as compared to the tonsil-derived stem cells (Negative control: a porous filter (membrane insert provided with a neurite outgrowth assay kit) was coated with BSA and N1E-115 cells were cultured in DMEM (+ 20 µg/mL gentamicin). Positive control: a porous filter was coated with laminin and N1E-115 cells were cultured in DMEM (+ 20 µg/mL gentamicin + 1 mg/mL BSA). NRPC and T-MSC groups: a porous filter was coated with BSA and N1E-115 cells were cultured in a culture of NRPCs or T-MSCs).

### Example 8. Cytokine array assay of neuronal regeneration-promoting cells

The expression of 507 cytokines was analyzed in T-MSC-1-1 and T-MSC-1-2, which showed the most prominent myelination in Example 4, and the neuronal regeneration-promoting cells differentiated therefrom.

The target cells were cultured for analysis of the cytokines. The target cells were seeded in a flask and cultured for 3-4 days. When the target cells filled 80% or more of the area of the flask, the culture medium was removed and the target cells were washed twice with DPBS. After the washing, the culture medium was replaced with DMEM (Dulbecco's phosphate-buffered saline) not containing FBS (fetal bovine serum), cytokines, etc. in order to rule out the effect of cytokines. The culture of the target cells was collected after culturing for 30 hours.

The collected culture was centrifuged at 3,600 rpm for 30 minutes. The supernatant was transferred to a centrifugal tube equipped with a cellulose membrane and concentrated by centrifuging at 3,600 rpm for 20 minutes. After the centrifugation, the conditioned medium that passed through the separation membrane was discarded and the culture of the same amount was added. The centrifugation was continued until the volume of the concentrated culture was decreased to 1 mL or below, and the concentrated culture was quantified by Bradford assay. The concentrated culture was adjusted to a final concentration of 1 mg/mL by mixing with DMEM.

A membrane coated with antibodies capable of detecting 507 cytokines (cytokine array kit, RayBiotech, USA) was reacted for 30 minutes by treating with a blocking buffer. After removing the blocking buffer remaining on the membrane and replacing with the concentrated culture, the membrane was reacted overnight in a refrigerator. The membrane was washed 7 times with a washing buffer. After adding a HRP-conjugated streptavidin solution, the membrane was reacted at room temperature for 2 hours. After removing the HRP-conjugated streptavidin solution, the membrane was washed 7 times with a washing buffer. After the washing, the membrane was soaked with an ECL (enhanced chemiluminescence) reagent and the expression of cytokines was confirmed using an imaging device.

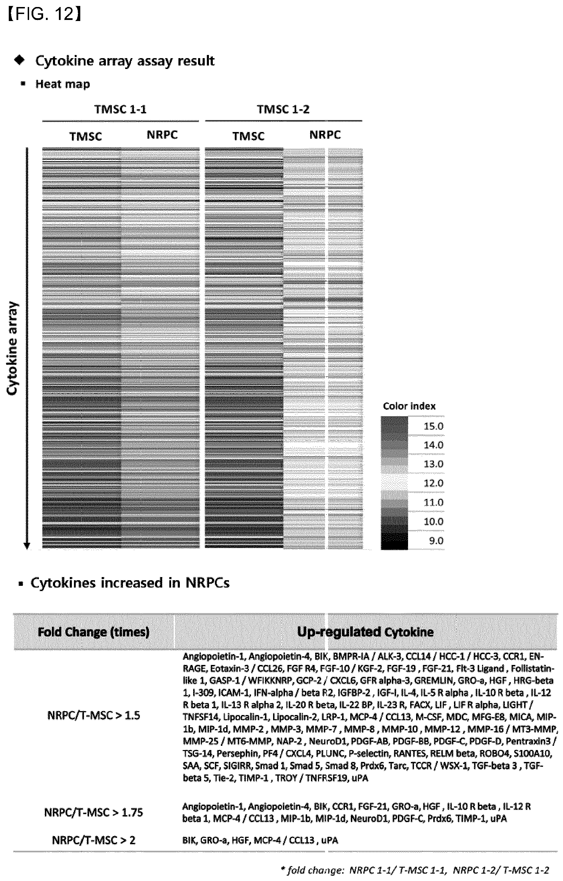

The result of comparing the expression of cytokines in the neuronal regeneration-promoting cells using heat maps is shown in FIG. 12. As shown in FIG. 12, the neuronal regeneration-promoting cells and the tonsil-derived mesenchymal stem cells showed different expression patterns.

The cytokines the expression of which has increased in the mesenchymal stem cells and neuronal regeneration-promoting cells derived from T-MSC-1-1 and T-MSC-1-2 are shown in FIG. 12.
- 1.5 fold or more: angiopoietin-1, angiopoietin-4, BIK, BMPR-IA / ALK-3, CCL14 / HCC-1 / HCC-3, CCR1, EN-RAGE, eotaxin-3 / CCL26, FGF R4, FGF-10 / KGF-2, FGF-19, FGF-21, Flt-3 Ligand, follistatin-like 1, GASP-1 / WFIKKNRP, GCP-2 / CXCL6, GFR alpha-3, GREMLIN, GRO-a, HGF , HRG-beta 1, I-309, ICAM-1, IFN-alpha / beta R2, IGFBP-2 , IGF-I, IL-4, IL-5 R alpha , IL-10 R beta, IL-12 R beta 1, IL-13 R alpha 2, IL-20 R beta, IL-22 BP, IL-23 R, FACX, LIF , LIF R alpha, LIGHT / TNFSF14, lipocalin-1, lipocalin-2, LRP-1, MCP-4 / CCL13, M-CSF, MDC, MFG-E8, MICA, MIP-1b, MIP-1d, MMP-2, MMP-3, MMP-7, MMP-8, MMP-10, MMP-12, MMP-16 / MT3-MMP, MMP-25 / MT6-MMP, NAP-2, NeuroD1, PDGF-AB, PDGF-BB, PDGF-C, PDGF-D, pentraxin3 / TSG-14, persephin, PF4 / CXCL4, PLUNC, P-selectin, RANTES, RELM beta, ROBO4, S100A10, SAA, SCF, SIGIRR, Smad 1, Smad 5, Smad 8, Prdx6, Tarc, TCCR / WSX-1, TGF-beta 3, TGF-beta 5, Tie-2, TIMP-1, TROY / TNFRSF19, uPA
- 1.75 fold or more: angiopoietin-1, angiopoietin-4, BIK, CCR1, FGF-21, GRO-a, HGF, IL-10 R beta , IL-12 R beta 1, MCP-4 / CCL13 , MIP-1b, MIP-1d, NeuroD1, PDGF-C, Prdx6, TIMP-1, uPA
- 2 fold or more: BIK, GRO-a, HGF, MCP-4 / CCL13, uPA

To conclude, the inventors of the present disclosure have prepared neuronal regeneration-promoting cells from tonsil- and adipose-derived mesenchymal stem cells and have investigated their expression pattern through CD marker assay. In addition, they have identified the neuronal regeneration effect of the neuronal regeneration-promoting cells. This suggests that the tonsil issues that have been discarded as medical wastes can be used to prepare cells having neuronal regeneration effect. The neuronal regeneration-promoting cells of the present disclosure can be utilized variously in the field of neuronal regeneration.

Although the specific exemplary embodiments of the present disclosure have been described, those having ordinary knowledge in the art can modify and change the present disclosure variously through the addition, change, deletion, etc. without departing from the scope of the present disclosure defined by the appended claims.

## Claims

1. A method for screening neuronal regeneration-promoting cells having neuronal regeneration activity from cells differentiated from mesenchymal stem cells, comprising:
a step of screening cells in which one or more marker selected from a group consisting of CD121a, CD106 and CD112 is up-regulated from among the differentiated cells as compared to mesenchymal stem cells before differentiation.

2. A method for screening neuronal regeneration-promoting cells having neuronal regeneration activity from cells differentiated from mesenchymal stem cells, comprising:
a step of screening cells in which one or more marker selected from a group consisting of CD26 and CD141 is down-regulated from among the differentiated cells as compared to mesenchymal stem cells before differentiation.

3. The method for screening neuronal regeneration-promoting cells according to claim 1 or 2, wherein the mesenchymal stem cells are derived from the tonsil or fat.

4. The method for screening neuronal regeneration-promoting cells according to claim 1 or 2, wherein the differentiated cells are differentiated from neurospheres formed by culturing mesenchymal stem cells.

5. The method for screening neuronal regeneration-promoting cells according to claim 1 or 2, wherein the neuronal regeneration activity comprises the myelination of peripheral nerves.

6. Neuronal regeneration-promoting cells screened by the screening method of claim 1 or 2, differentiated from tonsil-derived mesenchymal stem cells, the neuronal regeneration-promoting cells having the following characteristics:
a) the expression of the markers CD121a, CD106 and CD112 is up-regulated as compared to the tonsil-derived mesenchymal stem cells; and
b) the expression of the markers CD26 and CD141 is down-regulated as compared to the tonsil-derived mesenchymal stem cells.

7. The neuronal regeneration-promoting cells according to claim 6, wherein, in the neuronal regeneration-promoting cells, the expression of the marker CD121a is up-regulated by 30% or more as compared to the tonsil-derived mesenchymal stem cells.

8. The neuronal regeneration-promoting cells according to claim 6, wherein, in the neuronal regeneration-promoting cells, the expression of the marker CD106 is up-regulated by 5% or more as compared to the tonsil-derived mesenchymal stem cells.

9. The neuronal regeneration-promoting cells according to claim 6, wherein, in the neuronal regeneration-promoting cells, the expression of the marker CD112 is up-regulated by 10% or more as compared to the tonsil-derived mesenchymal stem cells.

10. The neuronal regeneration-promoting cells according to claim 6, wherein, in the neuronal regeneration-promoting cells, the expression of the marker CD26 is down-regulated by 5% or more as compared to the tonsil-derived mesenchymal stem cells.

11. The neuronal regeneration-promoting cells according to claim 6, wherein, in the neuronal regeneration-promoting cells, the expression of the marker CD141 is down-regulated by 5% or more as compared to the tonsil-derived mesenchymal stem cells.

12. A pharmaceutical composition for preventing or treating a neurological disease, comprising the neuronal regeneration-promoting cells according to claim 6 as an active ingredient, and a pharmaceutically acceptable carrier.

13. The pharmaceutical composition according to claim 12, wherein the neurological disease is one or more disease selected from a group consisting of Charcot-Marie-Tooth neuropathy, diabetic peripheral neuropathy, spinal cord injury, amyotrophic lateral sclerosis, carpal tunnel syndrome, infantile paralysis, leprosy, muscular dystrophy, polymyositis and myasthenia gravis.

14. The pharmaceutical composition according to claim 12, wherein the neuronal regeneration activity comprises the myelination of peripheral nerves.

15. The neuronal regeneration-promoting cells according to claim 6 for use in a method of treating a neurological disease.

## Patentansprüche

1. Verfahren zum Screenen von neuronalen regenerationsfördernden Zellen mit neuronaler Regenerationsaktivität aus Zellen, differenziert von mesenchymalen Stammzellen, umfassend:
einen Schritt des Screenens von Zellen, bei dem ein oder mehrere Marker, ausgewählt aus einer Gruppe bestehend aus CD121a, CD106 und CD112 hochreguliert ist, unter den differenzierten Zellen im Vergleich zu mesenchymalen Stammzellen vor der Differenzierung.

2. Verfahren zum Screenen von neuronalen regenerationsfördernden Zellen mit neuronaler Regenerationsaktivität aus Zellen, differenziert von mesenchymalen Stammzellen, umfassend:
einen Schritt des Screenens von Zellen, bei dem ein oder mehrere Marker, ausgewählt aus einer Gruppe bestehend aus CD26 und CD141herunterreguliert ist, unter den differenzierten Zellen im Vergleich zu mesenchymalen Stammzellen vor der Differenzierung.

3. Verfahren zum Screenen von neuronalen regenerationsfördernden Zellen nach Anspruch 1 oder 2, wobei die mesenchymalen Stammzellen aus der Mandel oder Fett stammen.

4. Verfahren zum Screenen von neuronalen regenerationsfördernden Zellen nach Anspruch 1 oder 2, wobei die differenzierten Zellen aus Neurosphären differenziert sind, gebildet durch Kultivierung mesenchymaler Stammzellen.

5. Verfahren zum Screenen von neuronalen regenerationsfördernden Zellen nach Anspruch 1 oder 2, wobei die neuronale Regenerationsaktivität die Myelinisierung peripherer Nerven umfasst.

6. Neuronale regenerationsfördernde Zellen, gescreent durch das Screening-Verfahren nach Anspruch 1 oder 2, differenziert von aus Mandeln stammenden mesenchymalen Stammzellen, wobei die neuronalen regenerationsfördernden Zellen folgende Charakteristika haben:
a) die Expression der Marker CD121a, CD106 und CD112 ist im Vergleich zu den aus Mandeln stammenden mesenchymalen Stammzellen hochreguliert; und
b) die Expression der Marker CD26 und CD141 ist im Vergleich zu den aus Mandeln stammenden mesenchymalen Stammzellen herunterreguliert.

7. Neuronale regenerationsfördernde Zellen nach Anspruch 6, wobei in den neuronalen regenerationsfördernden Zellen die Expression des Markers CD12la im Vergleich zu den aus Mandeln stammenden mesenchymalen Stammzellen um 30 % oder mehr hochreguliert ist.

8. Neuronale regenerationsfördernde Zellen nach Anspruch 6, wobei in den neuronalen regenerationsfördernden Zellen die Expression des Markers CD106 im Vergleich zu den aus Mandeln stammenden mesenchymalen Stammzellen um 5 % oder mehr hochreguliert ist.

9. Neuronale regenerationsfördernde Zellen nach Anspruch 6, wobei in den neuronalen regenerationsfördernden Zellen die Expression des Markers CD112 im Vergleich zu den aus Mandeln stammenden mesenchymalen Stammzellen um 10 % oder mehr hochreguliert ist.

10. Neuronale regenerationsfördernde Zellen nach Anspruch 6, wobei in den neuronalen regenerationsfördernden Zellen die Expression des Markers CD26 im Vergleich zu den aus Mandeln stammenden mesenchymalen Stammzellen um 5 % oder mehr herunterreguliert ist.

11. Neuronale regenerationsfördernde Zellen nach Anspruch 6, wobei in den neuronalen regenerationsfördernden Zellen die Expression des Markers CD141 im Vergleich zu den aus Mandeln stammenden mesenchymalen Stammzellen um 5 % oder mehr herunterreguliert ist.

12. Pharmazeutische Zusammensetzung zur Vorbeugung oder Behandlung einer neurologischen Erkrankung, umfassend die neuronalen regenerationsfördernden Zellen nach Anspruch 6 als Wirkstoff und einen pharmazeutisch akzeptablen Träger.

13. Pharmazeutische Zusammensetzung nach Anspruch 12, wobei die neurologische Erkrankung eine oder mehrere Erkrankung(en) ist/sind, ausgewählt aus einer Gruppe bestehend aus Charcot-Marie-Tooth-Neuropathie, diabetischer peripherer Neuropathie, Rückenmarksverletzung, amyotropher lateraler Sklerose, Karpaltunnelsyndrom, Kinderlähmung, Lepra, Muskeldystrophie, Polymyositis und Myasthenia gravis.

14. Pharmazeutische Zusammensetzung nach Anspruch 12, wobei die neuronale Regenerationsaktivität die Myelinisierung peripherer Nerven umfasst.

15. Neuronale regenerationsfördernde Zellen nach Anspruch 6 zur Verwendung in einem Verfahren zur Behandlung einer neurologischen Erkrankung.

## Revendications

1. Procédé pour cribler des cellules favorisant la régénération neuronale présentant une activité de régénération neuronale provenant de cellules différenciées à partir de cellules souches mésenchymateuses, comprenant :
une étape de criblage de cellules dans lesquelles un ou plusieurs marqueurs choisis dans le groupe constitué par CD121a, CD106 et CD112 sont régulés à la hausse parmi les cellules différenciées en comparaison avec des cellules souches mésenchymateuses avant différenciation.

2. Procédé pour cribler des cellules favorisant la régénération neuronale présentant une activité de régénération neuronale provenant de cellules différenciées à partir de cellules souches mésenchymateuses, comprenant :
une étape de criblage de cellules dans lesquelles un ou plusieurs marqueurs choisis dans le groupe constitué par CD26 et CD141 sont régulés à la baisse parmi les cellules différenciées en comparaison avec des cellules souches mésenchymateuses avant différenciation.

3. Procédé pour cribler des cellules favorisant la régénération neuronale selon la revendication 1 ou 2, dans lequel les cellules souches mésenchymateuses dérivent de tonsille ou de graisse.

4. Procédé pour cribler des cellules favorisant la régénération neuronale selon la revendication 1 ou 2, dans lequel les cellules différenciées sont différenciées à partir de neurosphères formées par culture de cellules souches mésenchymateuses.

5. Procédé pour cribler des cellules favorisant la régénération neuronale selon la revendication 1 ou 2, dans lequel l'activité de régénération neuronale comprend la myélinisation de nerfs périphériques.

6. Cellules favorisant la régénération neuronale criblées par le procédé de criblage de la revendication 1 ou 2, différenciées à partir de cellules souches mésenchymateuses dérivées de tonsille, les cellules favorisant la régénération neuronale ayant les caractéristiques suivantes :
a) l'expression des marqueurs CD121a, CD106 et CD112 est régulée à la hausse en comparaison avec les cellules souches mésenchymateuses dérivées de tonsille ; et
b) l'expression des marqueurs CD26 et CD141 est régulée à la baisse en comparaison avec les cellules souches mésenchymateuses dérivées de tonsille.

7. Cellules favorisant la régénération neuronale selon la revendication 6, dans lesquelles, dans les cellules favorisant la régénération neuronale, l'expression du marqueur CD121a est régulée à la hausse de 30 % ou plus en comparaison avec les cellules souches mésenchymateuses dérivées de tonsille.

8. Cellules favorisant la régénération neuronale selon la revendication 6, dans lesquelles, dans les cellules favorisant la régénération neuronale, l'expression du marqueur CD106 est régulée à la hausse de 5 % ou plus en comparaison avec les cellules souches mésenchymateuses dérivées de tonsille.

9. Cellules favorisant la régénération neuronale selon la revendication 6, dans lesquelles, dans les cellules favorisant la régénération neuronale, l'expression du marqueur CD112 est régulée à la hausse de 10 % ou plus en comparaison avec les cellules souches mésenchymateuses dérivées de tonsille.

10. Cellules favorisant la régénération neuronale selon la revendication 6, dans lesquelles, dans les cellules favorisant la régénération neuronale, l'expression du marqueur CD26 est régulée à la baisse de 5 % ou plus en comparaison avec les cellules souches mésenchymateuses dérivées de tonsille.

11. Cellules favorisant la régénération neuronale selon la revendication 6, dans lesquelles, dans les cellules favorisant la régénération neuronale, l'expression du marqueur CD141 est régulée à la baisse de 5 % ou plus en comparaison avec les cellules souches mésenchymateuses dérivées de tonsille.

12. Composition pharmaceutique pour prévenir ou traiter une maladie neurologique, comprenant les cellules favorisant la régénération neuronale de la revendication 6 en tant que principe actif, et un véhicule pharmaceutiquement acceptable.

13. Composition pharmaceutique selon la revendication 12, dans laquelle la maladie neurologique est une ou plusieurs maladies choisies dans le groupe constitué par une neuropathie de Charcot-Marie-Tooth, une neuropathie périphérique diabétique, une lésion de la moelle épinière, une sclérose latérale amyotrophique, un syndrome du canal carpien, une paralysie infantile, une lèpre, une dystrophie musculaire, une polymyosite et une myasthénie grave.

14. Composition pharmaceutique selon la revendication 12, dans laquelle l'activité de régénération neuronale comprend une myélinisation de nerfs périphériques.

15. Cellules favorisant la régénération neuronale selon la revendication 6, pour une utilisation dans le traitement d'une maladie neurologique.
